**Europäisches Patentamt**

**European Patent Office**

**Office européen des brevets**

(11) Veröffentlichungsnummer: **0 150 465**
A2

# EUROPÄISCHE PATENTANMELDUNG

(21) Anmeldenummer: **84116024.5**

(51) Int. Cl.⁴: **A 61 M 16/00**

(22) Anmeldetag: **21.12.84**

(30) Priorität: **12.01.84 DE 3400872**

(43) Veröffentlichungstag der Anmeldung: **07.08.85**
**Patentblatt 85/32**

(84) Benannte Vertragsstaaten: **AT CH DE FR GB IT LI**

(71) Anmelder: **Krüger, Christian, Prof. Dr. med.,**
**Curtiusstrasse 4, D-2400 Lübeck (DE)**

(72) Erfinder: **Krüger, Christian, Prof. Dr. med.,**
**Curtiusstrasse 4, D-2400 Lübeck (DE)**

(74) Vertreter: **Wilcken, Thomas, Dipl.-Ing. et al,**
**Musterbahn 1, D-2400 Lübeck (DE)**

(54) **Instrument zum Freihalten der oberen Atemwege und zur Durchführung von Intubationen.**

(57) Die Erfindung betrifft ein Instrument zum Freihalten der oberen Atemwege und zur Durchführung von Intubationen, bei dem ein einzuführender Tubus über einen als Führung dienenden Kanal mit seinem distalen Ende bis in die Luftröhre bewegt werden kann. Der Kanal ist als eine im Instrumentenschaft verlaufende Rinne ausgebildet, während das distale Instrumentenende aus einem Kopf besteht, vor dem die Rinne endet und der beim Einführen des Instrumentes gegen den Kehlkopf zur Anlage zu bringen ist. Der Instrumentenkopf wird somit als Anschlag die längstmögliche Einführstrecke begrenzen.

EP 0 150 465 A2

0150465

- 1 -

Anmelder: Prof. Dr. med. Christian Krüger,
Curtiusstraße 4, 2400 Lübeck


Instrument zum Freihalten der oberen Atemwege
und zur Durchführung von Intubationen


Die Erfindung betrifft ein Instrument zum Freihalten der oberen Atemwege und zur Durchführung von Intubationen, bei dem ein einzuführender Tubus über einen als Führung dienenden Kanal mit seinem distalen Ende bis in die Luftröhre bewegt wird.

Die orotracheale Intubation wird u.a. bei Fällen akuter Luftnot zur Schaffung eines freien Atemweges durch den in die Luftröhre eingeführten Tubus angewendet. Dabei wurde vor allem bei der anfänglichen Anwendung dieser Methode der Tubus "blind" bzw. "taktil" eingeführt. Erst die Entwicklung verschiedener Laryngoskope führte zu der heute gebräuchlichen Methode der Intubation unter Sicht.

Die Intubation mittels eines Laryngoskopes ist von einem geübten Arzt normalerweise ohne Komplikationen und auch relativ schnell durchführbar. Nachteilig ist es jedoch, daß der Patient speziell gelagert werden und der Arzt Bewegungsfreiheit am Kopf des Patienten haben muß. Die hierzu erforderlichen Vorbereitungen bringen einen entsprechenden Zeitaufwand mit

sich. Außerdem haben ungeübte Ärzte nicht selten Mühe, das Instrument richtig einzuführen und den Larynx "optisch einzustellen", so daß beispielsweise Verletzungen der vorderen Schneidezähne vorkommen können. Außerdem stehen auch die relativ teuren Laryngoskope nicht immer zur Verfügung. Schließlich machen diese Instrumente regelmäßig eine Wartung und Sterilisierung nach Gebrauch erforderlich.

Aus diesen Gründen hat es im Laufe der Zeit immer wieder Versuche gegeben, geeignete Methoden zum "taktilen" Einführen des Tubus zu finden, obwohl dabei ohne Beobachtung gearbeitet werden muß. So wurde schon vorgeschlagen, den Tubus mittels eines Mandrins einzuführen. Diese Methode konnte sich jedoch nicht durchsetzen. Allein die transnasale Intubation hat sich bis heute für spezielle Indikationen als taktile Methode behaupten können, die allerdings auch einige Erfahrung des Arztes voraussetzt und insbesondere insofern problematisch ist, als es nicht immer gelingen wird, den Tubus gleich genau und ohne Verletzungsgefahr durch den Rachenraum in die Luftröhre zu schieben.

Die Aufgabe der Erfindung besteht in der Schaffung eines Intubationsinstrumentes, das auch von ungeübten Ärzten und selbst von Laien nach entsprechender Einweisung sicher, schnell und gefahrlos angewendet werden kann. Außerdem soll das Instrument in der Herstellung billig sein, so daß es auch als Artikel zum nur einmaligen Gebrauch geeignet ist und somit u.a. bei der Notfall-Intubation eine entsprechend große Verbreitung finden kann.

Zur Lösung dieser Aufgabe wird das eingangs erwähnte Instrument erfindungsgemäß so ausgebildet, daß der Kanal als eine im Instrumentenschaft verlaufende Rinne ausgebildet ist und das distale Instrumentenende aus einem Kopf besteht, vor dem die Rinne endet und der beim Einführen des Instrumentes gegen den Kehlkopf zur Anlage zu bringen ist und als Anschlag die längstmögliche Einführstrecke begrenzt.

In Atemstellung liegt der Kehlkopf direkt an der Pharynxwand an und wandert beim Schluckakt auf einer bogenförmigen Linie nach vorn und oben, wobei er sich von der Pharynxwand entfernt. Umgekehrt wird durch eine induzierte und nach unten gerichtete Bewegung der Kehlkopf enger an den Pharynx gepreßt. Diesen anatomischen Gegebenheiten entsprechend wird also das vollständig eingeführte Instrument mit seinem distalen Kopf gegen den Kehlkopf anliegen, diesen teilweise umfassen und nach unten drücken. Gleichzeitig wird dabei der Instrumentenkopf zwischen dem Kehlkopf und dem Pharynx eingeklemmt. Diese Position muß in fester Relation zu den übrigen Abmessungen des Kehlkopfes erhalten bleiben, was vor allem dadurch sichergestellt werden kann, daß der Instrumentenkopf am distalen Ende mittig ausgekehlt ist, wodurch der Kehlkopf auch teilweise seitlich umfaßt wird. Außerdem kann der Instrumentenkopf auf der Rückseite eine axial verlaufende Auskehlung haben, wodurch sich der Kopf eng an den hier etwas nach vorn vorspringenden Pharynx anpassen wird. Dies hat zur Folge, daß der Instrumentenkopf einerseits den Kehlkopf beim Einführen des Instrumentes in der Medianlinie nicht passieren und andererseits nicht seitlich ausscheren wird, so daß eine größtmögliche Sicherheit dafür gegeben ist, daß sich das Instrument in der richtigen Position befinden wird, sobald man beim Vorgang des Einführens schließlich auf einen entsprechend großen Widerstand trifft.

Der Kanal wird durch die nach vorn offene, vor dem Instrumentenkopf endende Rinne eines im Querschnitt im wesentlichen halbkreisförmigen und biegsamen, im übrigen jedoch formstabilen Schaftes gebildet, wobei die Rinne an ihren Längsseiten in Gleitflächen übergehen sollte. Außerdem soll die Rinne am distalen Ende mit einer Rampe versehen sein oder in eine Rampe mit einer vom Rinnengrund ansteigenden Rampenfläche übergehen. Bei richtig eingeführtem Instrument kann dann der vorab in die Rinne eingelegte Tubus weiter distalwärts vorgeschoben und über die auf den Larynxraum des Patienten gerichtete Rampe gefahrlos und sicher bis in die Luftröhre geschoben werden.

Weitere Merkmale der Erfindung ergeben sich aus den Patentansprüchen, auf die hier zur Vermeidung von Wiederholungen Bezug genommen wird. Im übrigen wird die Erfindung nachfolgend anhand einiger Ausführungsbeispiele erläutert, die in der anliegenden Zeichnung schematisch und vereinfacht dargestellt sind. Es zeigen:

| Figur 1 | eine Seitenansicht eines Instrumentes nach der Erfindung, |
|---|---|
| Figur 2 | eine Aufsicht auf das Instrument nach Figur 1, |
| Fig. 3 - 6 | verschiedene Schnitte entsprechend den Schnittlinien III-III, IV-IV, V-V und VI-VI in Figur 2, |
| Figur 7 | das durch den Mund- und Rachenraum eingeführte Instrument nach den Figuren 1 bis 6, |
| Figur 8 | eine Aufsicht auf die vordere Seite einer anderen Ausführungsform des Instrumentes, |
| Figur 9 | eine Rückansicht des in Figur 8 dargestellten Instrumentes, |
| Figur 10 | einen Querschnitt durch das Instrument gemäß der Schnittlinie X-X in Figur 9, |
| Fig.11-13 | die Anwendung des Instrumentes nach den Figuren 8 bis 10 in drei verschiedenen Phasen, |
| Figur 14 | das in den Figuren 9 bis 13 dargestellte Instrument, jedoch mit einer anderen Ausbildung im proximalen Bereich, und |
| Figur 15 | einen Querschnitt durch das Instrument nach Figur 14 gemäß der Schnittlinie XV-XV. |

Das Instrument besteht im wesentlichen aus dem Schaft 1 und dem distalen Kopf 2. Der auch beim Biegen im Querschnitt formstabil bleibende Schaft hat eine längsverlaufende, nach vorn offene und vor dem Kopf 2 endende Rinne 3, die in allen dargestellten Beispielen im Querschnitt etwa halbkreisförmig ist und an ihren beiden Längsseiten in Gleitflächen 4, 5 und distal in eine Rampe 6 mit einer vom Rinnengrund 3a ansteigenden Rampenfläche übergeht.

Beim Instrument gemäß den Figuren 1 bis 7 hat der Kopf 2 im wesentlichen die Form einer Schale, einer Gabel oder eines Löffels, wobei die konkave Fläche 2a des Kopfes bei Anwendung des Instrumentes nach vorn gerichtet ist und beim Einführen des Instrumentes gegen den Kehlkopf zur Anlage gebracht werden soll, wie es noch später im einzelnen erläutert wird. Dabei bedeutet der Ausdruck "vorn", daß die hiermit bezeichneten Teile des Instrumentes dem vor dem Patienten stehenden Arzt zugewandt sind, während die "hinteren" Teile vom Arzt abgewandt sind. Mit anderen Worten ausgedrückt bedeutet dies, daß man beispielsweise in den Figuren 2 und 8 das Instrument von vorn sieht, während man das Instrument in Figur 9 von hinten betrachtet.

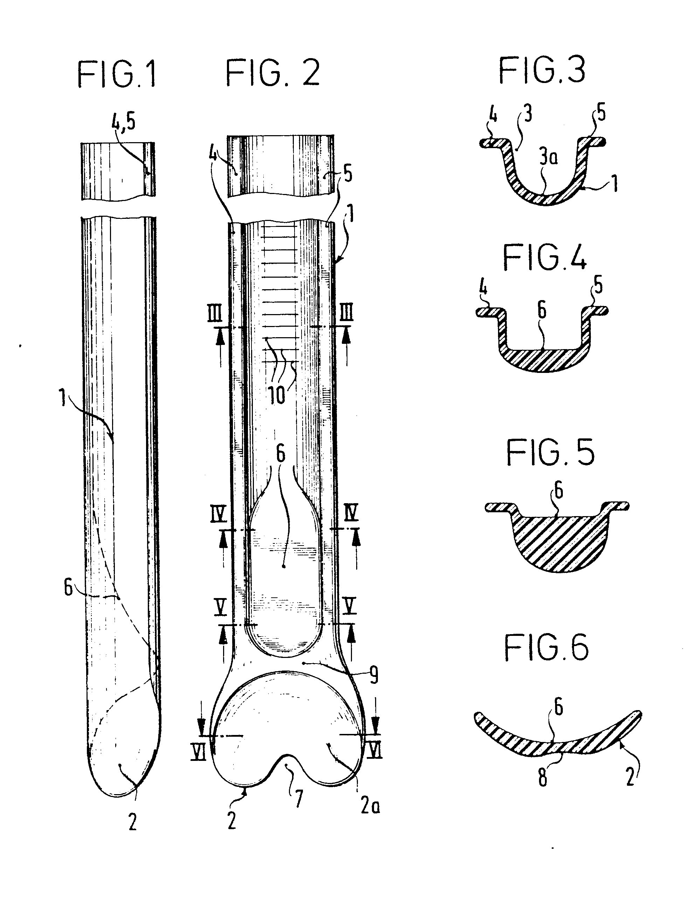

Bei der Ausführungsform nach den Figuren 1 bis 7 hat der Kopf 2 am freien Ende eine mittig angebrachte Auskehlung 7, so daß dort der Kopf zwei zum Randbereich hin dünner werdende Lappen bildet. Weiterhin hat der Kopf auf der Rückseite eine axial verlaufende Auskehlung 8. Im übrigen sind der Kopf 2 und die Rampe 6 durch einen quer zwischen ihnen verlaufenden Steg 9 getrennt, dessen Fläche der Darstellung gemäß in gleicher Ebene wie die Gleitflächen 4, 5 liegen oder auch hierzu nach hinten bzw. gemäß Figur 1 nach links versetzt angeordnet sein kann.

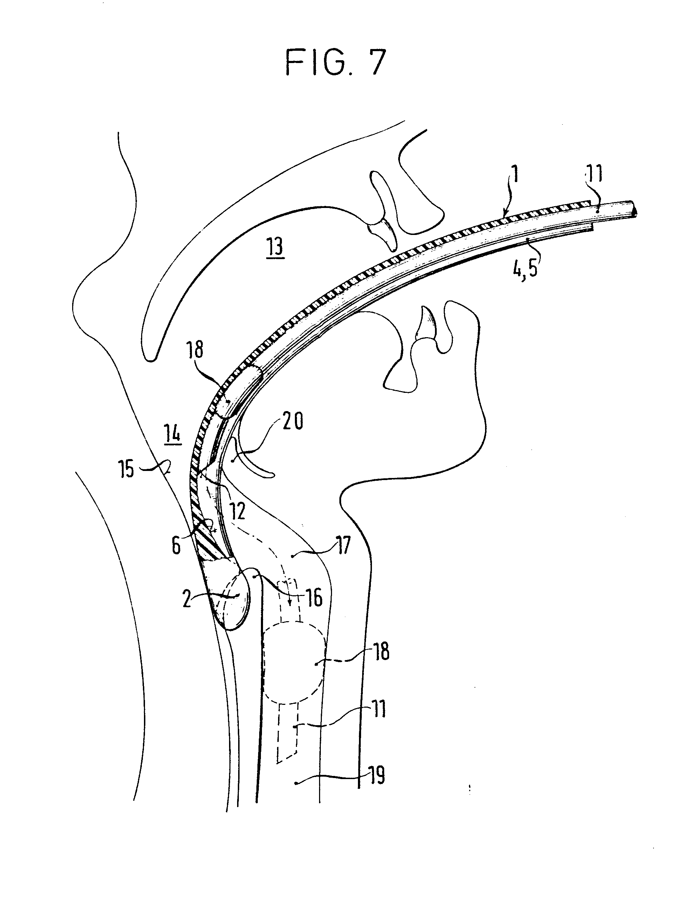

Das Instrument kann zwar einen geraden Verlauf haben, möglich ist es aber auch, daß man das Instrument bzw. dessen Schaft 1 den anatomischen Gegebenheiten des Mund- und Rachenraumes entsprechend gebogen herstellt, also etwa von vornherein die in Figur 7 gezeigte oder eine etwas geringere Krümmung wählt, wodurch verständlicherweise das Einführen des Instrumentes erleichtert werden könnte. Außerdem kann der Schaft 1 auf der Rückseite im Bereich des Krümmungsscheitels aufgeschlitzt sein. Solche in Figur 2 dargestellten Schlitze 10 ermöglichen ein leichteres Biegen des Schaftes im Sinne einer größeren Krümmung und somit besseren Anpassung an den Verlauf des Überganges vom Mund- zum Rachenraum.

Nachfolgend wird das Einführen eines Tubus 11 beschrieben. Der Tubus wird zunächst in die Rinne 3 des Schaftes so eingelegt, daß sein distales Ende möglichst direkt vor der Rampe 6 liegt. Dabei soll die durch den Schrägschnitt gebildete distale Endfläche 12 des Tubus 11 mit der ovalen Öffnung nach vorn zeigen (Figur 7) und in dieser Position fixiert gehalten werden, damit beim späteren Einführen des Tubus in die Luftröhre keine Verletzungen durch das spitze Tubusende auftreten können.

Wie schon eingangs erwähnt wurde, befindet sich der Patient zweckmäßigerweise in der Rückenlage, wobei die Halswirbelsäule durch entsprechende Neigung des Kopfes nach hinten leicht überstreckt werden sollte, da dann das Instrument aufgrund der geringeren erforderlichen Krümmung besser eingeführt werden kann. Das Instrument wird von Hand durch den Mundraum 13 und weiter durch den Rachenraum 14 geschoben, bis der Kopf 2 und der angrenzende gekrümmte Teil des Schaftes 1 an der hinteren Pharynxwand 15 anliegt. Bei diesem Vorgang sorgen die Gleitflächen 4, 5 dafür, daß das Instrument leicht und ungehindert über die Zunge und die distalwärts angrenzenden Teile im Rachenraum gleiten kann.

Anschließend wird der Zungenboden über die vordere Zahnreihe nach unten und vorn gedrückt, um dabei schließlich das Instrument so weit einzuführen, daß der Kopf 2 gemäß Figur 7 gegen den Kehlkopf 16 anliegt, die von hinten und oben teilweise mit seiner Fläche 2a umfaßt und im übrigen zwischen dem Kehlkopf 16 und der hinteren Pharynxwand 15 eingeklemmt wird. Dabei liegt in der hinteren Auskehlung 8 des Kopfes 2 ein Bereich der an dieser Stelle nach vorn vorspringenden Pharynxwand, so daß der Kopf 2 auch gegen eine seitliche Bewegung gesichert ist. Im gleichen Sinne wirkt auch die Auskehlung 7.

Da es hierbei vorkommen kann, daß der Kehlkopf 16 etwas verformt wird und hierdurch das freie Lumen eingeengt wird, sollte das Instrument zur Entlastung des Kehlkopfes im gegebenen Fall um eine gewisse Strecke wieder proximalwärts zurückgezogen werden.

Im übrigen kann bei diesem Vorgang der Arzt auch außerdem mit der freien Hand den Kehlkopf von außem am Hals des Patienten umfassen und sich von der richtigen Position des Instrumentenkopfes 2 überzeugen. Bei dieser Position ist die Rampe 6 mit ihrer Schrägfläche auf den Larynxraum 17 ausgerichtet, so daß jetzt der Tubus 11 in der Rinne 3 des Schaftes 1 distalwärts bewegt werden kann, bis das distale Tubusende einschließlich des am Tubusumfang befestigten Ballons 18 über die Rampe 6 in die Luftröhre 19 verschoben ist (Figur 7). Dann kann der Ballon in bekannter Weise über einen nicht weiter gezeigten und zum proximalen Instrumentenende führenden Schlauch aufgeblasen werden, damit er sich eng an die Luftröhre anlegt. Im Anschluß daran kann das Instrument wieder herausgezogen werden, während der Tubus 11 durch den aufgeblasenen Ballon in der Luftröhre fixiert verbleibt.

Der Sinn und Zweck einer Intubation dieser Art sind allgemein

bekannt, so daß hier nur darauf hingewiesen werden soll, daß über den Tubus 11 der Patient beispielsweise künstlich beatmet werden kann oder daß über den Tubus 11 Sekret entnommen oder Instrumente, Heilmittel oder dergleichen in den Respirationstrakt eingeführt werden können.

Von allgemeiner Bedeutung bei der Dimensionierung des Instrumentes ist es, daß die Öffnung der Rinne 3 nicht breiter sein sollte, als die Epiglottis 20, da die Gleitflächen 4, 5 beim Einführen des Instrumentes auf der Epiglottis aufliegen sollen. Weiterhin sollte der freie Durchmesser der Rinne 3 so gewählt werden, daß der Tubus 11 vollständig in der Rinne aufgenommen wird und nicht nach außen über die Gleitflächen hinaus vorsteht. Dies schließt aber nicht die Möglichkeit aus, daß man dem Tubus 11 dennoch einen größeren Durchmesser gibt, so daß man im Prinzip auf die Gleitflächen verzichten kann, da dann der nach vorn gerichtete Umfang des Tubus die Funktion der Gleitflächen übernehmen wird.

Außerdem soll der Steg 9 so schmal sein, daß auch die kürzeste anatomisch vorkommende Epiglottis beim Einführen des Instrumentes noch abgehoben wird. Weiterhin darf die Rampe 6 nur so steil sein, daß auch Tuben 11 mit großem Durchmesser die notwendige Sekundärbiegung beim Übergang vom Larynxraum 17 in die Luftröhre 19 vollziehen können und sich mit dem distalen Ende nich im Larynxraum verfangen. Schließlich kann bei einem Instrument dieser Art der Kopf 2 leichter biegsam als der Schaft 1 sein, da sonst das Einführen des Instrumentes erschwert und die Gefahr von Verletzungen bestehen könnte.

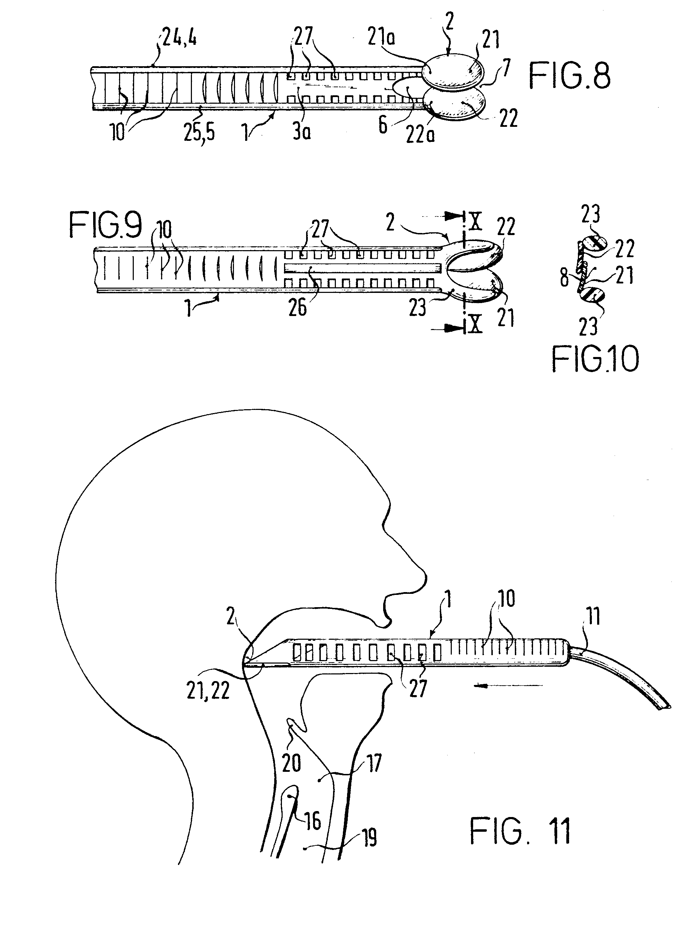

Die Figuren 8 bis 13 zeigen ein anderes Ausführungsbeispiel für ein Instrument nach der Erfindung. Soweit Teile dieses Instrumentes mit denen des vorher beschriebenen Beispieles in ihrer Art und Funktion übereinstimmen, wurden für solche

0150465

Teile gleiche Bezugsziffern verwendet.

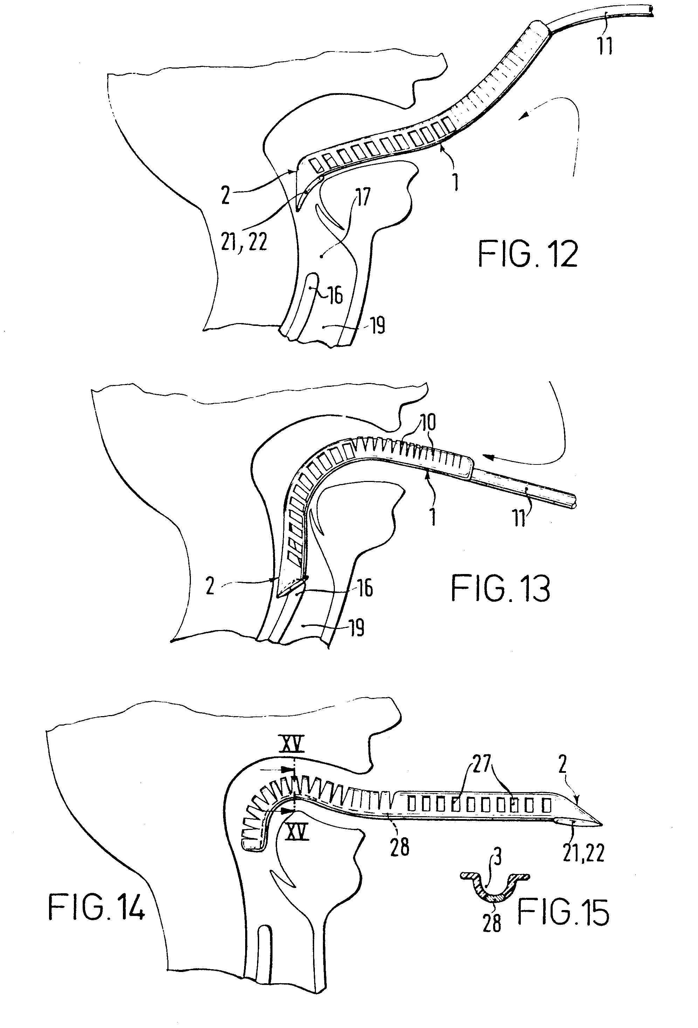

Der Instrumentenkopf 2 besteht aus zwei flexiblen Klappen 21, 22, die am äußeren Rand eines gabelförmigen Teiles 23 des Kopfes sitzen und sich gegenseitig überlappen. Diese Klappen gelangen beim Einführen des Instrumentes gegen den Kehlkopf 16 (Figur 13) zur Anlage und werden dabei nach hinten ausgelenkt, so daß sie den Kehlkopf teilweise umfassen werden.

Die proximalen Enden 21a, 22a der Klappen liegen in der Verlängerung der schräg vom Rinnengrund 3a distalwärts ansteigenden Flächen der Rampe 6, so daß sie durch das Ende des über die Rampe zu schiebenden Tubus 11 etwas nach vorn flexibel verbogen werden, wodurch zunächst die Tubusspitze und dann der Tubus selbst zwischen den geringfügig aufgespreizten Klappenenden zentriert geführt wird und nicht zur Seite hin ausweichen kann.

Die Figuren 11 bis 13 demonstrieren das Einführen des Instrumentes in drei hauptsächlichen Phasen. Um die Biegung des Kopfes 2 in der Einführungsphase gemäß Figur 12 zu erleichtern und quasi fernsteuern zu können, ist der Schaft 1 an seinen beiden Längsseiten mit Schienen 24, 25 versehen, die gleichzeitig die Funktion der vorerwähnten Gleitflächen 4, 5 übernehmen. Außerdem ist außen am Schaft 1 und dem Rinnengrund 3a gegenüberliegend eine weitere Schiene 26 angebracht. Diese drei Schienen können mit dem fertig vorbereiteten Schaft durch Verschweißung oder durch Kleben verbunden werden. Andererseits besteht natürlich auch die Möglichkeit, daß die Schienen gleich bei der Herstellung des Schaftes mit in das Schaftmaterial eingebettet werden.

Die drei Schienen verlaufen entweder bis an oder in den Bereich des Kopfes 2 und sind steifer als das relativ weiche Material des Schaftes. Wenn nun der Arzt das Instrument so weit wie in Figur 11 gezeigt eingeführt hat, kann er mit einer Hand die Abbiegung des Kopfes 2 gemäß Figur 12 herbeiführen, indem er z.B. mit der Handfläche einen distal gerichteten Druck auf die Schiene 26 und mit dem Daumen bzw. den Fingern einen proximal gerichteten Zug auf die Schienen 24, 25 ausübt.

Danach kann das Instrument leicht und ohne Verletzungsgefahr weiter bis in die Position nach Figur 13 geschoben werden, wobei vor Erreichen dieser Position die Zug- und Druckverhältnisse an den Schienen umgekehrt werden sollten, damit der Kopf 2 den Kehlkopf 16 sicher passieren und hinter den Kehlkopf gelangen kann.

Die hierbei zwangsläufig auftretenden Kräfte zur Verformung des Schaftmaterials werden reduziert, wenn der die Rinne bildende Teil des Schaftes 1 im Bereich der Schiene 26 mit fensterartigen Ausnehmungen 27 versehen ist, die sich in den beiden an den Rinnengrund 3a anschließenden Seitenwänden des Schaftes befinden. Außerdem kann die Beweglichkeit des Kopfes 2 im Verhältnis zum Schaft 1 in der Weise verbessert werden, daß der Kopf flexibel am Schaft angelenkt wird. Dies läßt sich beispielsweise so erreichen, daß der Querschnitt des Instrumentes am Übergang vom Schaft zum Kopf eingeschnürt oder kleiner gehalten wird als die sich hieran anschließenden Querschnitte.

Im übrigen hat der Schaft 1 auch bei diesem Ausführungsbeispiel Schlitze 10, und zwar zumindest im Bereich der einen beim Einführen des Instrumentes erforderlichen Krümmung (Figur 13), wobei die Schlitze das Schaftmaterial im Bereich des Rinnengrundes 3a trennen und sich auch bis in die beiden hieran anschließenden Seitenwände der Rinne bzw. des Schaftes erstrecken.

Wie bereits im Zusammenhang mit dem Ausführungsbeispiel nach den Figuren 1 bis 7 erwähnt wurde, kann der Arzt, sobald das Instrument in die in Figur 13 gezeigte Stellung geführt ist und ggf. noch eine Korrektur der erreichten Stellung durch weiteres Hineinschieben oder Herausziehen des Instrumentes durchgeführt wurde, den Tubus 11 über die Rampe 6 in den Raum 17 und weiter in die Luftröhre 19 schieben, so daß sich die etwa auch in Figur 7 gezeigte Situation ergibt und anschließend das Instrument vollständig wieder herausgezogen werden kann, während der Tubus 11 in seiner Lage verbleibt.

Wenn das Instrument nicht zur Durchführung von Intubationen verwendet werden soll, sondern nur zum Freihalten der oberen Atemwege dienen soll, wird das Instrument ohne Tubus in die in den Figuren 7 und 13 dargestellte Position gebracht, so daß die Atemwege zwangsläufig freigehalten werden und der Patient über freie Wege atmen kann, die einerseits durch den Instrumentenkanal und andererseits auch durch neben dem eingeführten Instrument entstandene Räume geschaffen sind.

Insofern zeigen schließlich auch die Figuren 14 und 15 eine relativ einfache Lösung, bei der auch das proximale Ende des Instrumentes zum Freihalten der oberen Atemwege nach Art eines an sich bekannten Guedel-Tubus benutzt werden kann, wenn sich im oder am Schaft 1 zumindest eine in Figur 14 gestrichelt angedeutete Einlage oder Auflage 28 befindet, welche den Schaft in der jeweils durch Biegen hergestellten Form (Figur 14) stabilisiert. Diese in das Schaftmaterial eingebettete Einlage oder am Schaft befestigte Auflage kann aus einer Schiene aus z.B. Weicheisen, Kupfer und dergleichen bestehen und sich wie dargestellt über den Bereich des Schaftes erstrecken, der auch mit den Schlitzen 10 versehen ist.

Bevor das so ausgebildete Instrument mit dem proximalen Ende eingeführt wird, kann der Arzt dieses Ende den anatomischen Gegebenheiten des Patienten entsprechend, ggf. auch unter Verwendung einer Schablone, biegen und dann durch den Mund bis in den Rachenraum einführen, um z.B. in akuten Notfällen einen Patienten vorläufig versorgen und erreichen zu können, daß die Atemwege freigehalten und nicht durch ein mögliches Zurückfallen der Zunge versperrt werden.

Die auf dem Markt befindlichen Tuben 11 haben meist ein distales Ende, das durch einen schräg zur Tubusachse geführten Schnitt hergestellt ist. Solch relative spitzen Tubusenden können beim Einführen leicht zu Verletzungen führen. Aus diesem Grunde erscheint es zweckmäßig, wenn man das distale Tubusende mit einem Gleitelement versieht, das mit Hilfe von distal her in den Tubus einzuführenden Drahtes oder sonstigen Verbindungselementes von außen in das Tubusende gezogen werden kann, um bei entsprechender Formgebung des Gleitelementes den asymmetischen Raum neben dem schräg angeschnittenen Tubusende auszugleichen.

Ein solches Gleitelement könnte beispielsweise die Form einer geschlossenen Schlaufe haben, die mit einem durch den Tubus geführten Draht in den Bereich des Tubusendes gezogen wird und sich gegen eine weitere Bewegung in den Tubus durch von außen einwirkende Kräfte sperrt, bei Zug am Draht aber einen kleineren Durchmesser erhält und sich leicht durch den Tubus wieder herausziehen läßt.

Versuche haben ergeben, daß in der Praxis mit beiden dargestellten und beschriebenen Instrumenten zufriedenstellend gearbeitet werden kann. Die Ausführungsform nach den Figuren 8 bis 10 könnte aber beim Einführen des Instrumentes gewisse größere Vorteile haben. Die Klappen 21, 22 sollen zwar aus relativ weichem Material bestehen, sie sollen aber dennoch ohne Verformung über die in den zur Verfügung stehenden Einführungskanal vorstehende Epiglottis gleiten und diese aus dem Kanal nach vorn wegdrücken können. Es muß also vermieden werden, daß die Epiglottis vom distalwärts bewegten Instrumentenkopf mit nach unten genommen wird.

Im übrigen werden sich die beiden Klappen 21, 22 bei Anlage gegen den Kehlkopf 16 (Figur 13) schräg nach hinten stellen und dabei den Kehlkopf seitlich sowie von oben und hinten umfassen, wobei die größtmögliche Auslenkung der flexiblen Klappen nach hinten durch einen Schlag so begrenzt werden soll, daß der Instrumentenkopf 2 nicht zu weit hinter den Kehlkopf 16 nach unten gleiten kann. Ein solcher Anschlag wird beim dargestellten Beispiel durch den gabelförmigen Teil 23 des Instrumentenkopfes gebildet.

Die Schienen 24, 25 und 26 müssen nicht unbedingt gesonderte Bauteile sein, die am Schaft 1 angebracht oder in dessen Material eingebettet sind. Es ergeben sich auch gleichwirkende "Schienen", wenn das Material des Schaftes an den beiden Längsseiten und am Rinnengrund bei längsgerichtetem Druck oder Zug steifer ist als das übrige Material an den Schaftseitenwänden, was sich meist schon durch die Anbringung der Ausnehmungen 25 in den Seitenwänden erreichen lassen wird.

Abschließend wird noch darauf hingewiesen, daß ein Schaft 1 mit einer offenen Rinne 3 zum einen das Einlegen des Tubus 11 und zum anderen auch das Herausziehen des Instrumentes erleichtern wird, nachdem der Tubus in die Luftröhre 19 vorgeschoben ist. Möglich ist es jedoch auch, den Schaft als Rohr auszubilden, das etwa bis zur Rampe 6 hin geschlossen ist, wobei es in diesem Fall allerdings zweckmäßig erscheint, das Rohr vorn längs aufzuschlitzen, damit sich das Rohr beim Herausziehen des Instrumentes im Bereich des Längsschlitzes aufweiten und den Tubus nach außen treten läßt.

0150465

Anmelder: Prof. Dr. med. Christian Krüger,
Curtiusstraße 4, 2400 Lübeck

Patentansprüche

1. Instrument zum Freihalten der oberen Atemwege oder zur
   Durchführung von Intubationen, bei dem ein einzuführender Tubus über einen als Führung dienenden Kanal mit
   seinem distalen Ende bis in die Luftröhre bewegt wird,
   dadurch gekennzeichnet, daß der Kanal als eine im Instrumentenschaft (1) verlaufende Rinne (3) ausgebildet
   ist und das distale Instrumentenende aus einem Kopf (2)
   besteht, vor dem die Rinne endet und der beim Einführen
   des Instrumentes gegen den Kehlkopf (16) zur Anlage zu
   bringen ist und als Anschlag die längstmögliche Einführstrecke begrenzt.

2. Instrument nach Anspruch 1, dadurch gekennzeichnet, daß
   die Rinne (3) am distalen Ende eine Rampe (6) bildet
   oder in eine Rampe (6) mit einer vom Rinnengrund (3a)
   ansteigenden Rampenfläche übergeht.

3. Instrument nach Anspruch 1 oder 2, dadurch gekennzeichnet,
   daß der Kopf (2) im wesentlichen schalen-, löffel- oder
   gabelförmig ausgebildet ist.

4. Instrument nach einem der Ansprüche 1 bis 3, dadurch gekennzeichnet, daß der Kopf (2) am distalen Ende eine
   mittig angeordnete Auskehlung (7) hat.

5. Instrument nach einem der Ansprüche 1 bis 4, dadurch gekennzeichnet, daß der Kopf (2) zwei sich gegenseitig überlappende Klappen (21, 22) aufweist, die bei Anlage gegen
   den Kehlkopf (16) flexibel nach hinten auslenkbar sind,
   und daß die proximalen Enden (21a, 22a) der Klappen in
   der Verlängerung der Rampenfläche liegen und eine zentrierende Führung für einen über die Rampe (6) distalwärts zu schiebenden Tubus (11) bilden.

6. Instrument nach einem der Ansprüche 1 bis 5, dadurch gekennzeichnet, daß der Kopf (2) flexibel am Instrumentenschaft (1) angelenkt ist.

7. Instrument nach einem der Ansprüche 1 bis 6, dadurch gekennzeichnet, daß die Rinne (3) an ihren beiden Längsseiten in ebene Gleitflächen (4, 5) übergeht.

8. Instrument nach Anspruch 7, dadurch gekennzeichnet, daß die beiden Gleitflächen (4, 5) durch Schienen (24, 25) gebildet werden, daß eine weitere Schiene (26) im Bereich des Rinnengrundes vorgesehen ist und daß die Schienen steifer als der Schaft sind.

9. Instrument nach einem der Ansprüche 1 bis 8, dadurch gekennzeichnet, daß der Schaft (1) zumindest im Bereich einer beim Einführen des Instrumentes erforderlichen Krümmung aufgeschlitzt ist und daß die Schlitze (10) das Schaftmaterial im Bereich des Rinnengrundes (3a) trennen und sich bis in die Seitenwände des Schaftes erstrecken.

10. Instrument nach einem der Ansprüche 1 bis 9, dadurch gekennzeichnet, daß der die Rinne (3) bildende Teil des Schaftes (1) an beiden sich an den Rinnengrund (3a) anschließenden Seitenwänden mit Ausnehmungen (27) versehen ist.

11. Instrument nach einem der Ansprüche 1 bis 10, dadurch gekennzeichnet, daß der Schaft (1) am proximalen Bereich wenigstens eine durch Biegen plastisch verformbare Schiene (28) als Einlage oder Auflage hat, welche den Schaft in der jeweils durch Biegen hergestellten Form stabilisiert.

0150465

12. Instrument nach einem der Ansprüche 1 bis 11, dadurch
    gekennzeichnet, daß für das distale Ende des Tubus (11)
    ein Gleitelement vorgesehen ist, das mit Hilfe eines
    von distal her in den Tubus einzuführenden Drahtes von
    außen in das Tubusende ziehbar ist, um den asymmetrischen Raum neben dem schräg angeschnittenen Tubusende
    auszugleichen, und der nach Gebrauch durch den Tubus
    proximalwärts herausziehbar ist.

FIG.1    FIG. 2    FIG.3

FIG.4

FIG. 5

FIG.6

FIG. 7

0150465

FIG.8

FIG.9

FIG.10

FIG. 11

FIG. 12

FIG. 13

FIG. 14

FIG. 15